# EUROPEAN PATENT APPLICATION

(11) **EP 2 246 095 A1**
(43) Date of publication of application: **03.11.2010**
(21) Application number: 09713110.6
(22) Date of filing: 16.02.2009
(51) Int. Cl.: A61N 5/06, A45D 26/00, A61H 23/02, A61H 33/12

(54) **LIGHT IRRADIATION BEAUTY APPLIANCE**

(30) Priority: 18.02.2008 JP 2008036324
(71) Applicant: Panasonic Electric Works Co., Ltd, Kadoma-shi Osaka 571-8686 (JP)
(72) Inventor: UCHIDA, Satoshi, Kadoma-shi Osaka 571-8686 (JP)
(74) Representative: Sherrard-Smith, Hugh
(86) International application number: PCT/JP2009/052481
(87) International publication number: WO 2009/104530

(57) **Abstract**

A light irradiation cosmetic device (1) that performs even light irradiation and eliminates the Influence of hair follicle grime. The light irradiation cosmetic device (1) includes a light irradiation unit (5), which irradiates skin (H) with light to perform skin beautification, and a removal unit (6), which suctions a portion of the skin (H) that undergoes light Irradiation before the light irradiation unit (5) performs light irradiation to remove hair follicle grime from the skin.

## Description

The present invention relates to a light irradiation cosmetic device that irradiates skin with light to obtain a cosmetic effect.

A light irradiation cosmetic device known in the prior art obtains a cosmetic effect for the skin by irradiating the skin with light to improve blood circulation and metabolism. For example, patent document 1 describes a light irradiation cosmetic device including a condenser lens that gathers laser light of a semiconductor laser diode and emits the gathered light toward the skin surface. The energy of the laser light irradiation removes abnormal pigment calls that are scattered in the epidermis and dermis and improves sebum secretion for skin beautification.

With regard to skin beautification, in addition to skin health as described above, it is important that grime be removed from hair follicles. Such hair follicle grime includes, for example, horny plugs formed when the sebum in a hair follicle or near the outlet of the hair follicle mixes with grime suspended in the air. The oxidation of horny plugs darkens the skin and traps the sebum. This may result in blackheads. When using the above-described light irradiation cosmetic device, the darkened hair follicle grime excessively absorbs light energy. This may result in the skin being irradiated with an uneven amount of light energy.

The prior art example of the light irradiation cosmetic device described in patent document 1 includes a suction means (compressor) to suction the portion of the skin irradiated with the laser light. However, the document does not include any particular description on the removal of such hair follicle grime. It is thus presumed that the purpose of the skin auction is to stabilize the irradiation state (distance) of the laser light. Even if the skin suction were to allow for the removal of hair follicle grime, the portion irradiated with the laser light is the same as the suctioned portion. Thus, the removed hair follicle grime would collect on the condenser lens and obstruct even light irradiation. Accordingly, the prior art does not provide a to the above-described problem, and improvements may be made from this aspect.
Patent Document 1: Japanese Laid-Opeh Patent Publication No. 2000-202045

The present invention provides a light irradiation cosmetic device that eliminates the influence of hair follicle grime and allows for even light emission.

One aspect of the present invention is a light irradiation cosmetic device including a light irradiation unit that irradiates skin with light to perform skin beautification. A removal unit that removes hair follicle grime from the skin by suctioning a portion of the skin that will undergo light irradiation before the light irradiation unit performs light irradiation.
Fig. 1 is a schematic cross-sectional view entirely showing a light irradiation cosmetic device according to a first embodiment;
Fig. 2 is a schematic cross-sectional view showing the main part of a light irradiation cosmetic device according to a second embodiment;
Fig. 3 is a schematic diagram showing a mist spray unit;
Figs. 4A and 4B are explanatory diagrams showing the effect of mist spraying;
Fig. 5 is a schematic cross-sectional view showing the main part of a light irradiation cosmetic device according to a third embodiment;
Fig. 6 is a side view showing the outer appearance of the main part of a light irradiation cosmetic device according to a fourth embodiment; and
Fig. 7 is a front view showing the outer appearance of the main part of a light irradiation cosmetic device according to the fourth embodiment.

### First Embodiment

A light irradiation cosmetic device 1 according to a first embodiment of the present invention will now be discussed with reference to the drawings.

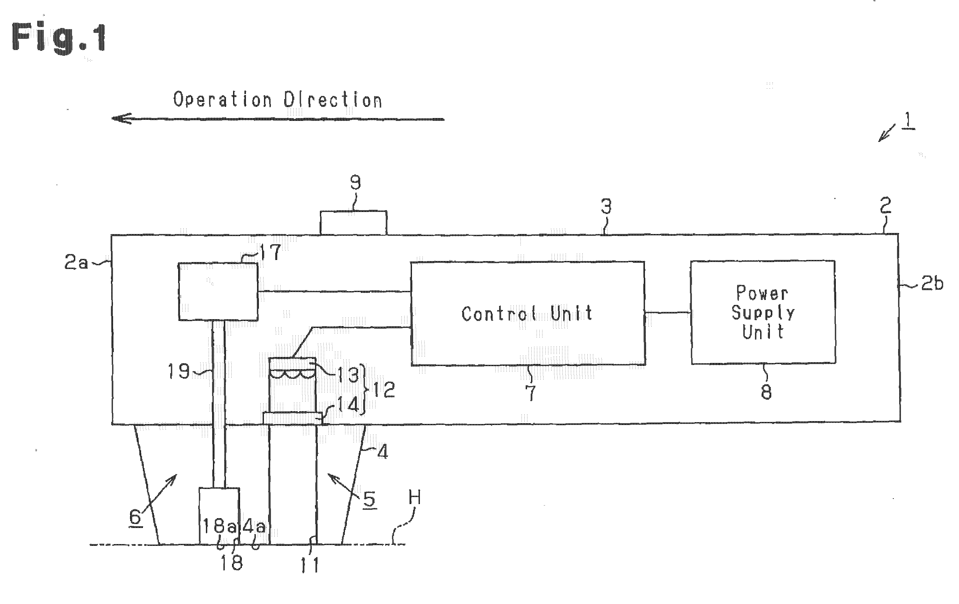

Fig. 1 is a schematic cross-seofional view entirely showing the light irradiation cosmetic device 1 according to the present embodiment. As shown in the drawing, the light irradiation cosmetic device 1 of the present embodiment is a so-called hand-held light irradiation cosmetic device and includes a tubular housing 2 (device body) formed to have a size allowing for a user to hold and operate it. A grip 3 is formed on the periphery of the housing 2 at the portion held by the user. Further, the housing 2 has a distal end 2a from which a tubular head 4 projects in a direction substantially orthogonal to the axis of the housing 2.

In the present embodiment, the head 4 includes a light irradiation unit 5, which irradiates the user's skin with light to perform skin beautification, and a skin suction unit 6, which serves as a removal means or removal unit for removing hair follicle grime from the skin by applying suction force to the user's skin. Further, the housing 2 accommodates a control unit 7, which supplies power to and controls the operation of the light irradiation unit 5 and skin suction unit 6, and a power supply unit 8. In the present embodiment, a warning unit, which is not illustrated, is arranged In the housing 2, and a switch 9 is arranged on the periphery of the housing 2 near the grip 3. In the present embodiment, when the switch 9 is operated, the control unit 7 starts supplying drive power to the light irradiation unit 5 and the skin suction unit 6, that is, starts light irradiation and skin suction.

In detail, the head 4 of the present embodiment includes a light irradiation opening 11, which extends along the axis of the head 4. Further, the housing 2 includes a light source unit 12 arranged at a position corresponding to the light irradiation opening 11. The light source unit 12 of the present embodiment includes a light source 13, which emits light when driven by the control unit 7, and a light transmissive cover 14, which closes the end of the light irradiation opening 11 that is closer to the housing 2. In the present embodiment, the light source 13 is capable of outputting a low-output non-coherent light, and the cover 3 functions as a lens and a wavelength filter. The light irradiation unit 5 of the present embodiment outputs irradiation light of the light source unit 12 through the light irradiation opening 11 so that the user's skin H, which is arranged facing toward the distal end 4a of the head 4, is Irradiated with light for skin beatification. The light irradiation unit 5 is not limited to the structure shown in Fig. 1 and may have another structure that is known to one skilled In the

The skin suction unit 6 includes a pump 17, which generates suction force, a cup 18, which applies the suction force to the user's skin H, and a tube 19, which connects the pump 17 and the cup 18. The cup 18 is arranged in the head 4 in a state in which its open end 18a is flush with the distal end 4a of the head 4. Further, the pump 17 accommodated in the housing 2 is controlled by the control unit 7 so as to suction the air in the cup 18 through the tube 19. More specifically, the skin suction unit 6 of the present embodiment decreases the pressure in the cup 18 in a state in which the open end 18a is pressed against the skin H so that its suction force is applied to the user's skin H. This structure removes hair follicle grime from the portion the cup 18 is pressed against. The skin suction unit 6 (removal unit) is not limited to the structure shown in Fig. 1 and may have another structure that is known to one skilled in the art.

In the present embodiment, with respect to the axial direction of the housing 2 (lateral direction as viewed in Fig. 1), the cup 18 that forms the skin suction unit 6 is arranged further toward the distal end 2a (left side as viewed in the drawing) from the light irradiation opening 11, which forms the light irradiation unit 5. An operation direction is set for the light irradiation cosmetic device 1 of the present embodiment so that the light irradiation cosmetic device 1 moves along the skin surface from the side of the basal and 2b toward the side of the distal end 2a in the axial direction of the housing 2 in a state in which the cup 18 of the head 4 is pressed against the user's skin H.

More specifically, in the light irradiation cosmetic device 1 of the present embodiment, the user holds the housing 2, presses the cup 18 of the skin suction unit 6 against the portion of the skin H that is to undergo cosmetic treatment, and turns on the switch 9 to start skin suction with the skin suction unit 6 and light irradiation with the light irradiation unit 5. After performing skin suction for a certain period, the housing 2 is moved in the predetermined operation direction so that the portion that has undergone skin suction next undergoes light irradiation.

The light irradiation cosmetic device 1 of the present embodiment sequentially repeats skin suction for a certain period and movement of the housing 2 in the predetermined operation direction, This simplifies and facilitates the series of cosmetic treatments that perform light irradiation on portions that have undergone skin suction. This prevents uneven light irradiation, which would be caused by hair follicle grime, and allows for cosmetic treatments to be effectively performed. Further, hair follicles from which grime has been removed are literally in a "state in which hair follicles are open" and extremely sensitive, Thus, it is preferable that cosmetic treatments be performed as soon as possible. In this aspect, the present embodiment readily treats such hair follicles that have undergone grime removal. These two treatments act in a synergetic manner with each other and obtain superior cosmetic effects.

### Second Embodiment

A light Irradiation cosmetic device 21 according to a second embodiment of the present invention will now be discussed with reference to the drawings. To facilitate description, the same reference numerals are given to those components that are the same as the corresponding components of the first embodiment, and such components will not be described.

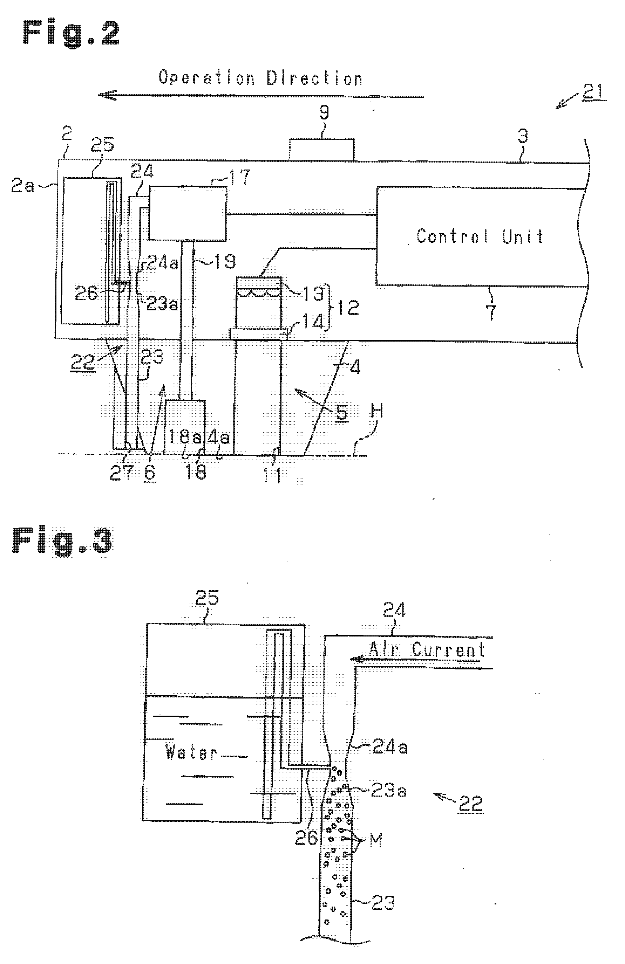

Fig. 2 is a schematic cross-sectional view showing the main part of the light irradiation cosmetic device 21 according to the second embodiment. In addition to the structure of the light irradiation cosmetic device 1 according to the first embodiment, the light irradiation cosmetic device 21 of the present embodiment includes a mist spray unit 22, which sprays mist onto the user's skin H.

In the head 4 of the present embodiment includes a mist passage 23 having a basal end 23a, which is connected to a blower tube 24 that guides air current to the mist passage 23. As shown in Fig. 3, In the present embodiment, the basal end 23a of the mist passage 23 and an outlet 24a of the blower tube 24 are each tapered so that the diameter gradually decreases. A water supply tube 26, which extends from a water tank 25 arranged in the housing 2, is coupled to the part at which the mist passage 23 and the blower tube 24 are coupled.

More specifically, the air current sent into the blower tube 24 when the pump 17 is operated is accelerated when passing through the tapered outlet 24a, As a negative pressure, or a venturi effect, is produced at the coupling part of the mist passage 23 and the tube 24. This generates mist M from the water drawn out of the water supply tube 26. The mist M moves through the mist passage 23.

As shown in Fig. 2, the mist passage 23 has a distal end forming a mist discharge port 27. In the present embodiment, the mist discharge port 27 is located next to the cup 18, which forms the skin suction unfit 6. In detail, the mist discharge port 27 is arranged further toward the distal end 2a (leftward in the drawing) from the cup 18 in the axial direction of the housing 2 (lateral direction as viewed in Fig. 2). In the same manner as in the first embodiment, the user holds the horsing 2 and turns on the switch 9 in a state in which the cup 18 of the skin auction unit 6 is pressed against the portion of the skin H that is to undergo cosmetic treatment. This starts mist spraying with the mist spray unit 22 described above in addition to skin suction and light irradiation.

During use, in the same manner as in the first embodiment, after performing skin suction for a certain period, the light irradiation cosmetic device 31 of the present embodiment is moved along the skin surface in the predetermined operation direction, or in the axial direction of the housing from the side of the basal end 2b toward the side of the distal end 2a, while the cup 18 of the skin suction unit 6 is pressed against the user's skin H. By sequentially repeating skin suction for a certain period and movement of the housing 2 in the predetermined operation direction, the spraying of mist with the mist spray unit 22 is performed in a simplified and facilitated manner before the skin suction and light irradiation. The mist spray unit 22 is not limited to the structure shown in Figs. 2 and 3 and may have another structure that is known to one skilled in the art.

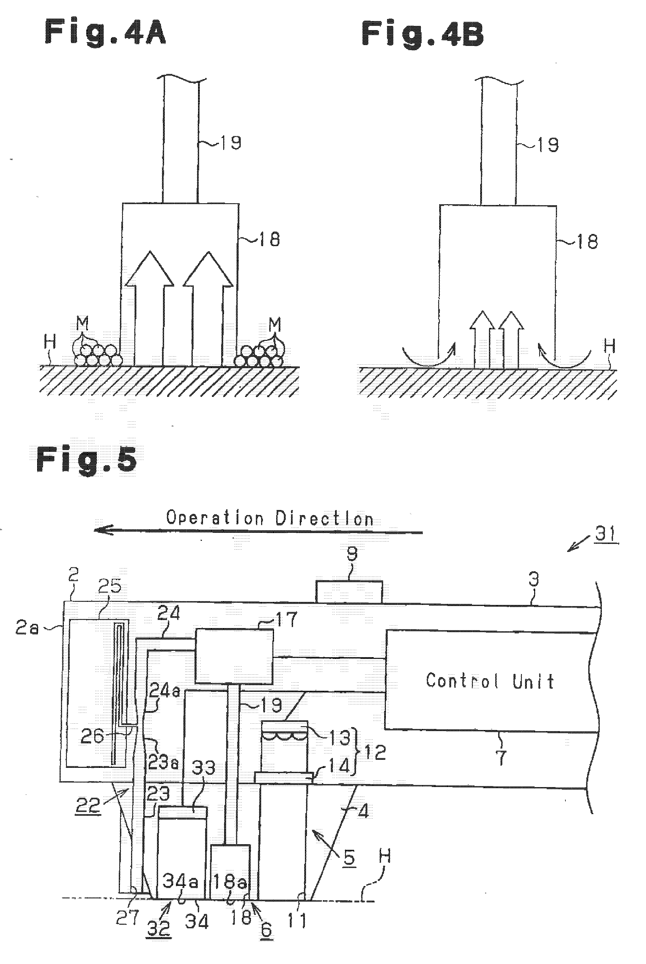

With the structure of the present embodiment, a synergetic effect may be expected from the effect of the moisture applied to the skin H by the mist spray and the effect of the removal of hair follicle grime. In particular, by performing mist spraying on a portion of the skin H before performing skin suction on this portion, the mist M sprayed to the skin H eliminates gaps between the skin H and the cup 18, which forms the skin suction unit 6, and thereby increase the suction force (refer to Figs. 4A and 4B). As a result, hair follicle grime is removed further effectively. Further, by performing mist spraying on a portio of the skin H before performing light irradiation on this portion, light irradiation may be performed in a state in which sufficient moisture is applied to the skin H. This prevents the light irradiation from advancing dry skin and further enhances the effect of the cosmetic treatment.

### Third Embodiment

A light irradiation cosmetic device 31 to to a third embodiment of the present invention will now be discussed with reference to the drawings. To facilitate description, the same reference numerals are given to these components that are the same as the corresponding components of the second embodiment, and such components will not be described.

Fig. 5 is a schematic cross-sectional view showing the main part of the light irradiation cosmetic device 31 according to the third embodiment, As shown in the drawing, in addition to the structure of the light irradiation cosmetic device 21 according to the second embodiment, the light irradiation cosmetic device 31 of the present embodiment includes an ultrasonic vibration unit 32, which applies ultrasonic vibration to the user's skin H.

The ultrasonic vibration unit 32 includes an ultrasonic vibrator 33, which generates ultrasonic vibration when supplied with drive power the control unit 7, and 7, and a horn 34, which transmits the ultrasonic vibration. The supply of drive power to the ultrasonic vibrator 33 from the control unit 7 is started by ting the switch 9. In the present embodiment, the ultrasonic vibration unit 32 is arranged between the mist discharge port 27, which forms the mist spray unit 22 and the cup 18, which forms the skin auction unit 6. More specifically, the ultrasonic vibration unit 32 is arranged in the head 4 so that the distal end 34a of the horn 34 is with the end 4a of the head 4. Further, in the some manner as in the first and second embodiments, the user holds The housing 2 and turns on the switch 9 in a state in which the cup 18 of the skin suction unit 6 and the horn 34 of the ultrasonic vibration unit 32 are pressed the portion of the skin H that is to undergo cosmetic treatment. This starts the application of ultrasonic vibration with the ultrasonic vibration unlit 32 described above in addition to skin suction, light irradiation, and mist spraying.

During in the same manner as in the first and second embodiments, after performing skin suction for a certain period, the light irradiation cosmetic device 31 of the present embodiment is moved the skin surface in the predetermined operation or in the axial direction of the housing 2 from the side of the basal end 2b toward the side of the distal end 2a, while the cup 18 of the skin suction unit 6 and the horn 34 of the ultrasonic vibration unit 32 are pressed against the user's skin H. By sequentially repeating skin in suction for a certain period and movement of the housing 2 in the predetermined operation direction, the application of ultrasonic vibration by the ultrasonic vibration unit 32 is performed in a simplified and facilitated manner before the skin suction and light irradiation. The ultrasonic vibration unit 32 (ultrasonic vibration application unit) is not limited to the structure shown in Fig. 5 and may have another structure that is known to one skilled In the art.

With the structure of the present embodiment, a synergetic effect may be expected from the massaging effect produced by the application of ultrasonic vibration and the effect of the removal of hair follicle grime. In particular, the application of ultrasonic vibration to a portion of the skin H before performing skin suction on this portion facilitates the removal of grime from the skin H in general, such as debris from the skin. This allows for further even light irradiation and further increases the cosmetic effect of the light irradiation.

### Fourth Embodiment

A light irradiation cosmetic device 41 according to a fourth embodiment of the present invention will now be discussed with reference to the drawings. To facilitate description, the same reference numerals are given to those components that are the same as the corresponding components of the third embodiment, and such components will not be described.

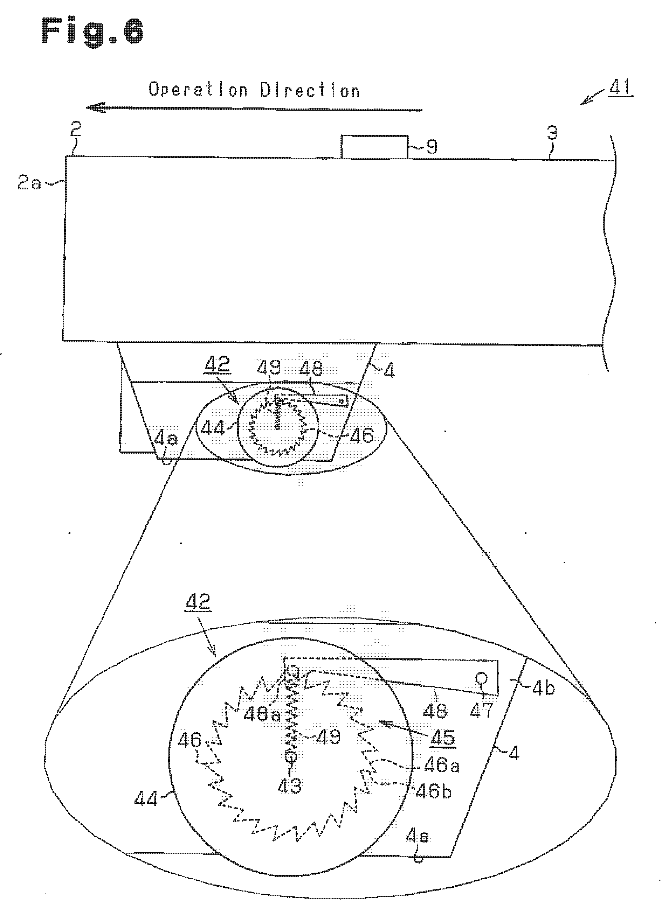

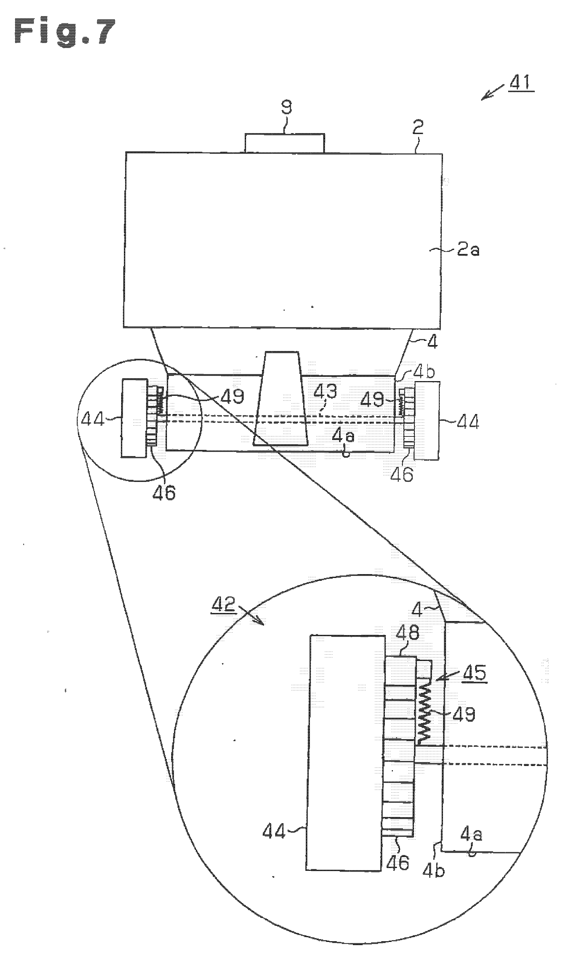

Fig. 6 is is a view showing the outer appearance of the main part of the light Irradiation cosmetic device 41 according to the fourth embodiment, and Fig. 7 is its front view. In addition to the structure of the light irradiation cosmetic device 31 according to the third embodiment, as shown in Figs, 6 and 7, the light irradiation cosmetic device 41 of the present embodiment includes a movement direction restriction device 42, which serves as a restriction unit that restricts movement of the housing 2 (and the head 4) in directions other than a predetermined operation direction, which is set beforehand.

In detail, in the present embodiment, the head 4 includes a rotary shaft 3, which extends through the head 4 in the widthwise direction of the head 4 (lateral direction as viewed in Fig. 7). The rotary shaft 43 has two ends on which are arranged a pair of assistance wheels 44. Each assistance wheel 44 is arranged sideward from the heat 4 in a state partially projecting from the distal end 4a of the head 4. Movement of the housing 2 (head 4) in the axial direction in a state pressed against the user's skin H rotates each assistance wheel 44 while obstructing, or restricting, movement of the housing 2 in the axial direction of the rotary shaft 43 with the projecting portion of each assistance wheel 44.

In the present embodiment, a rotation restriction mechanism 45 is incorporated in each of the assistance wheels 44 so that its rotation direction is restricted to one direction. More specifically, a gear 46 is fixed to an inner surface 44a (surface facing toward the side surface 4b of the head 4) of each assistance wheel 44 so that the gear 46 rotates integrally with the assistance wheel 44. The distal end of a stopper 48, the pivot axis of which is an axial pin projecting from the side surface 4b of the head 4, is engaged with the gear 46. In the present embodiment, a tension spring 49, which is hooked to the stopper 48 and the rotary shaft 43. urges the distal end of the stopper 48 in a direction pivoted toward the gear 46. In the present embodiment, the gears 46, the stopper 48, and the tension spring 49 form the rotation restriction mechanism 45.

More specifically, in the present embodiment, the teeth surface of the gear 46 has a surface 46a (surface In the counterclockwise direction as viewed in Fig. 6) facing toward the rotation direction when the housing 2 moves in the predetermined operation direction (toward the distal end 2a in the axial direction) that is a gradually sloped surface. In contrast, a surface 46b (surface in the clockwise direction as viewed in Fig. 6) facing toward the rotation direction when the housing 2 moves In a direction opposite to the predetermined operation direction is a steeply sloped surface. Further, a catch 48a arranged on the distal end of the stopper 48 has a shape that conforms to the teeth surface of the gear 46, that is, shaped to engage with the valleys of the gear 46.

In other words, when moving the housing 2 in the predetermined operation direction, movement of the housing 2 in the predetermined operation direction is permitted when the assistance wheels 44 are each rotated while lifting the catch 48a of the stopper 48, which is engaged with the gear 46. When moving the horsing 2 in a direction opposite to the predetermined operation direction, the catch 48a of the stopper 48 engaged with the gear 46 strikes the teeth surface, or the steeply sloped surface 46b. This restricts rotation of each auxiliary wheel 44 and thereby restricts movement of the housing 2 in the direction opposite to the predetermined operation direction.

In this manner, the Movement direction restriction device 42 restricts the movement direction of the housing 2 to an axial direction with the assistance wheels 44. Further, the movement direction restriction device 42 restricts the rotation direction of each assistance wheel 44 to one direction with the rotation restriction mechanism 45. This restricts movements in directions other than the preset operation direction. The movement direction restriction device 42 (restriction unit) is not limited to the structure shown in Figs. 6 and 7 and may have another structure that is known to one skilled in the art.

The structure of the present embodiment allows for the user to accurately operate the housing 2 in the predetermined operation direction in accordance with the restriction (guidance) of the movement direction restriction device 42. This prevents erroneous operations by the user and allows for the series of cosmetic treatments, such as the spraying of mist to the skin H, the application of ultrasonic vibration, akin suction, and light irradiation to be consecutively performed in an optimal order in a facilitated and easy manner. In addition, the assistance wheels 44 facilitate the operation for moving the housing 2 on the surface of the skin H.

Each of the embodiments discussed above may be modified as described below.

The predetermined operation direction of the housing 2 (cosmetic devices 1, 21, 31, and 41) is not limited to the direction from the side of the basal end 2b toward the side of the distal end 2a in the axial direction of the housing 2. Preferably, when changing the predetermined operation direction, the layout of the light irradiation unit 5 serving as a light irradiation means, the skin suction unit 6 serving as a removal means, the mist spray unit 22 serving as a mist spray means, and the ultrasonic vibration unit 32 serving as an ultrasonic vibration application means may be changed in accordance with the change in the operation direction.

Th mist discharge port 27 of the mist spray unit 22 does not have to be arranged toward the distal end 2a from the cup 18 of the skin suction unit 6 in the axial direction of the housing 2, Further, the horn 34 of the ultrasonic vibration unit 32 does not have to be arranged between the mist discharge port 27 of the mist spray unit 22 and the cup 18 of the skin suction unit 6. A cosmetic effect maybe expected due to the synergetic effect of the mist spray and application of ultrasonic vibration with light irradiation, However, It is preferable that the and the application of ultrasonic vibration be performed before the light irradiation and more preferably before the skin suction.

The third embodiment is based an the structure of the second embodiment but may be changed to a structure based on the first embodiment. More specifically, the third embodiment may have a structure in which the ultrasonic Vibration application means is combined with the light irradiation means and the removal means.

In the same manner, the fourth embodiment is based on the structure of the third embodiment but may be changed to a structure based on the first embodiment or second embodiment. More specifically, the fourth embodiment may have a structure in which the light irradiation means and the removal means is combined with the restriction means (movement direction restriction device 42). Alternatively, the fourth embodiment may have a structure in which the light irradiation means, the removal means, and the mist spray means are combined with the restriction means. Further, the fourth embodiment may have a structure in which the light irradiation means, the removal means, and the ultrasonic vibration application means are combined with the restriction means.

The assistance wheels 44 and each rotation restriction mechanism 45 (the gear 46, the stopper 48, and the tension spring 49), which restrict the rotation direction of the assistance wheel 44 to one direction form the movement direction restriction means serving as a restriction means. However, the restriction means is not limited to this structure and may have another structure.

The urging means that urges the stopper 48 toward the gear side may be a structure that uses an elastic member other than the tension spring 49.

In each of the above embodiments, the switch 9 is operated to activate each means that performs a cosmetic treatment (the light Irradiation unit 5, the skin suction unit 6, the mist spray unit 22, and the ultrasonic vibration unit 32). However, activation of these means is not particularly limited, and different operations may be performed to activate these means.

## Claims

1. A light irradiation cosmetic device comprising:
a light irradiation unit that irradiates skin with light to perform skin beautification; and
a removal unit that removes hair follicle grime from the skin by suctioning a portion of the skin that will undergo light irradiation before the light irradiation unit performs light irradiation.

2. The light irradiation cosmetic device according to claim 1, further comprising:
a device body that accommodates the removal unit and the light irradiation unit and is operated in a state pressed against the skin to move along a skin surface:
wherein the removal unit is arranged along a predetermined operation direction set for the device body and further from the light Irradiation unit in the predetermined operation direction.

3. The light irradiation cosmetic device according to claim 2, further comprising:
a restriction unit that restricts movement of the device body in a direction other than the predetermined operation direction.

4. The light irradiation cosmetic device according to any one of claims 1 to 3, further comprising:
a mist spray unit that sprays mist onto the skin.

5. The light irradiation cosmetic device according to claim 4, wherein the removal unit is arranged between the light irradiation unit and the mist spray unit.

6. The light irradiation cosmetic device according to any one of claims 1 to 3, further comprising:
an ultrasonic vibration application unit that applies ultrasonic vibration to the skin.

7. The light Irradiation cosmetic device according to claim 6, wherein the removal unit is arranged between the light irradiation unit and the ultrasonic vibration application unit.
